(19)

Europäisches
Patentamt
European
Patent Office
Office européen
des brevets

(11) **EP 2 151 426 A1**

(12) **EUROPEAN PATENT APPLICATION**
published in accordance with Art. 153(4) EPC

(43) Date of publication:
**10.02.2010 Bulletin 2010/06**

(21) Application number: **08752761.0**

(22) Date of filing: **15.05.2008**

(51) Int Cl.:
***C07C 29/149*** (2006.01)

(86) International application number:
**PCT/JP2008/058900**

(87) International publication number:
**WO 2008/149648 (11.12.2008 Gazette 2008/50)**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **06.06.2007 JP 2007149841**

(71) Applicant: **Ube Industries, Ltd.
Ube-shi
Yamaguchi 755-8633 (JP)**

(72) Inventors:
• **II, Hirofumi
Yamaguchi 755-8633 (JP)**
• **KURAFUJI, Toshio
Yamaguchi 755-8633 (JP)**
• **KAWAMURA, Yoshiki
Yamaguchi 755-8633 (JP)**

(74) Representative: **HOFFMANN EITLE
Patent- und Rechtsanwälte
Arabellastrasse 4
81925 München (DE)**

(54) **METHOD OF PRODUCING 1,5-PENTANEDIOL AND/OR 1,6-HEXANEDIOL**

(57)      An objective is to provide a process for preparing 1,5-pentanediol and/or 1,6-hexanediol comprising esterifying, with an alcohol such as methanol, ethanol, butanol, or 1,6-hexanediol, a mixture of carboxylic acids such as glutaric acid, adipic acid and 6-hydroxycaproic acid which are a by-product in preparation of cyclohexanone by oxidation of cyclohexane with oxygen or an oxygen-containing gas; and hydrogenating the resulting esterified product in the presence of a copper-containing catalyst, which process is an industrially suitable process for preparing 1,5-pentanediol and/or 1,6-hexanediol in a high yield while controlling deterioration of the catalyst. The above objective is achieved by hydrogenating the esterified product with a catalyst obtained by prereducing a copper-containing catalyst in an alcohol having an acid value (AV) of 0.5 mg KOH/g or less or an ester having an acid value (AV) of 0.5 mg KOH/g or less.

**EP 2 151 426 A1**

## Description

Technical Field

The present invention relates to a process for preparing 1,5-pentanediol and/or 1,6-hexanediol, more particularly, a process for preparing 1,5-pentanediol and/or 1,6-hexanediol using a prereduced copper-containing hydrogenation catalyst.

Background Art

As a process for preparing 1,6-hexanediol, Patent Document 1 describes a known process for preparing 1,6-hexanediol comprising generating a carboxylic acid compound such as adipic acid or oxycaproic acid by oxidation of cyclohexane; esterifying the carboxylic acid compound with an alcohol such as ethanol, butanol or 1,6-hexanediol: and hydrogenating the resulting esterified product in the presence of a catalyst. In this preparation process, 1,5-pentanediol can also be prepared.

A problem in this process for preparing 1,6-hexanediol is that when an esterified product having a high acid value (AV) is hydrogenated, the catalyst deteriorates and the rate of conversion to 1,6-hexanediol is decreased.

As a means for solving such a problem, Patent Document 2 describes a process for preparing 1,6-hexanediol comprising esterifying with an alcohol a carboxylic acid compound obtained by oxidation of cyclohexane; bringing a liquid mixture of the resulting esterified product into contact with a basic solid substance such as barium hydroxide to reduce the acid value (AV) of the mixture to 0.8 mg KOH/g or less; and hydrogenating the mixture in the presence of a catalyst.

Patent Document 3 describes that when a fatty acid ester is hydrogenated to prepare an alcohol, a reduction in activity of a copper-chromium catalyst that is a hydrogenation catalyst for the aliphatic ester can be inhibited by lowering the acid value of the fatty acid ester to 2 mg KOH/g or less. Specifically, Patent Document 3 describes a method in which a monohydric or polyhydric alcohol having 1 to 18 carbon atoms is allowed to be present in hydrogenation.

As described above, it is known that deterioration of a catalyst can be inhibited by adjusting the acid value of an esterified product. However, since the acid value of the whole of esterified product must be controlled, it is necessary to carry out a complicated operation such as an operation of adding an alcohol separately or bringing into contact with a basic solid substance. This is disadvantageous for an industrial preparation process.

As a process for preparing 1,6-hexanediol with a specified acid value (AV) of an esterified product, Patent Document 4 describes a process for preparing 1,6-hexanediol comprising esterifying a fraction containing adipic acid, oxycaproic acid and their oligomers as main components which are by-products obtained by separating s-caprolactone from crude ε-caprolactone obtained from reaction of an organic peracid with cyclohexanone; and hydrogenating the esterified product having an acid value (AV) of 10 mg KOH/g or less in presence of a catalyst.

With regard to prereduction of catalyst, Patent document 5 describes a process for preparing a copper-containing hydrogenation catalyst comprising liquid phase reduction of a copper-containing hydrogenation catalyst precursor in an aliphatic ester or aliphatic alcohol by supplying a hydrogen gas or a mixed gas of hydrogen and an inert gas in the presence of a solvent inert to copper oxide or metallic copper, and a process for preparing an alcohol comprising hydrogenating an ester using the catalyst. However, Patent Document 5 has no mention about the acid value (AV) and a suspended bed catalyst.

Patent Document 1: Japanese Patent Publication No. Sho 53-33567 (1978)
Patent Document 2: Japanese Unexamined Patent Publication No. Hei 3-34946 (1991)
Patent Document 3: Japanese Unexamined Patent Publication No. Hei 5-978 (1993)
Patent Document 4: Japanese Unexamined Patent Publication No. 2005-35974
Patent Document 5: Japanese Unexamined Patent Publication No. Hei 7-163880 (1995)
Patent Document 6: Japanese Patent No. 3161578
Non-Patent Document 1: Ullmann's Encyclopedia of Industrial Chemistry, 5. Ed, 1987, Vol.A8, S.2/9

Disclosure of the Invention

An objective of the present invention is to provide a process for preparing 1,5-pentanediol and/or 1,6-hexanediol comprising esterifying, with an alcohol such as methanol, ethanol, butanol or 1,6-hexanediol, a mixture of carboxylic acids such as glutaric acid, adipic acid and 6-hydroxycaproic acid which are a by-product in preparation of cyclohexanone by oxidation of cyclohexane with oxygen or an oxygen-containing gas; and hydrogenating the resulting esterified product in the presence of a copper-containing catalyst, which process is an industrially suitable process for preparing 1,5-pentanediol and/or 1,6-hexanediol in a high yield with deterioration of the catalyst controlled.

The present inventors have found that the above objective is achieved by hydrogenating the esterified product with a catalyst obtained by reducing (prereducing) a copper-containing catalyst in an alcohol having an acid value (AV) of 0.5 mg KOH/g or less or an ester having an acid value (AV) of 0.5 mg KOH/g or less. This finding has led to the completion of the present invention.

That is, the first invention relates to a process for preparing 1,5-pentanediol and/or 1,6-hexanediol, comprising esterifying monocarboxylic and dicarboxylic acids which are by-products in preparation of cyclohexanone by oxidation of cyclohexane; and hydrogenating the resulting esterified product in the presence of a copper-containing catalyst reduced (prereduced) in the presence of an alcohol having an acid value (AV) of 0.5 mg KOH/g or less or/and an ester having the same acid value.

The second invention relates to the process for preparing 1,5-pentanediol and/or 1,6-hexanediol according to the first invention, wherein the esterified product has an acid value (AV) of 4 mg KOH/g or less.

The third invention relates to the process for preparing 1,5-pentanediol and/or 1,6-hexanediol according to the first invention, wherein the alcohol is an aliphatic alcohol having 1 to 6 carbon atoms.

The fourth invention relates to the process for preparing 1,5-pentanediol and/or 1,6-hexanediol according to the first invention, wherein the copper-containing catalyst is a catalyst containing copper oxide or a composite metal oxide composed of copper oxide and zinc oxide.

The fifth invention relates to the process for preparing 1,5-pentanediol and/or 1,6-hexanediol according to the first invention, wherein the copper(0) (0-valent copper) of the reduced (prereduced) copper-containing catalyst has a crystallite size of 150 Å or less.

The sixth invention relates to a copper-containing catalyst for hydrogenation wherein the copper(0) has a crystallite size of Effects of Invention 150 A or less.

Effects of Invention

In the process for preparing 1,5-pentanediol and/or 1,6-hexanediol according to the present invention comprising esterifying, with an alcohol such as methanol, ethanol, butanol or 1,6-hexanediol, a mixture of carboxylic acids such as glutaric acid, adipic acid and 6-hydroxycaproic acid which are a by-product in preparation of cyclohexanone by oxidation of cyclohexane; and hydrogenating the resulting esterified product in the presence of a copper-containing catalyst, the desired 1,6-hexanediol and/or 1,5-pentanediol can be prepared at a high conversion rate while controlling deterioration of the catalyst.

Brief Description of the Drawings

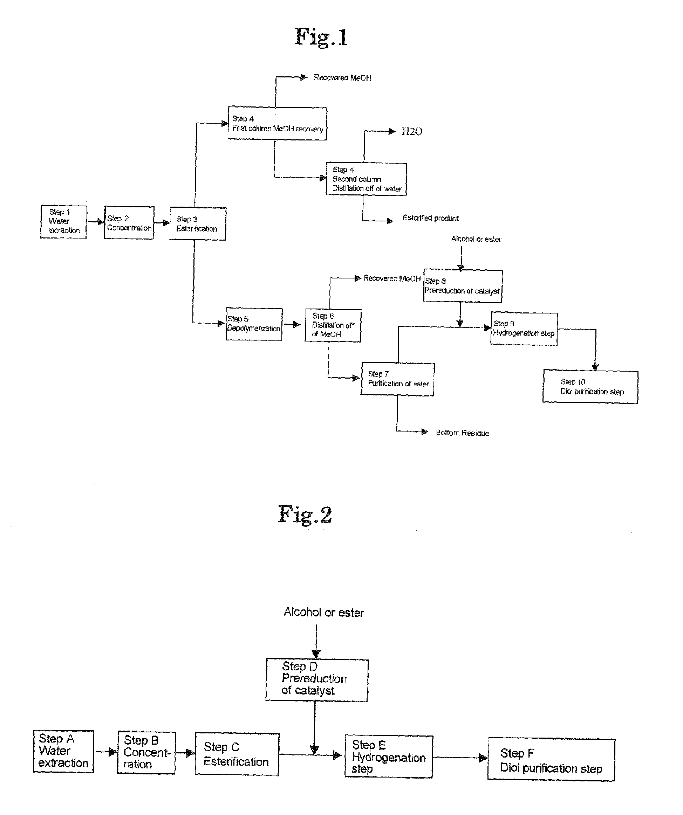

Figure 1 is a schematic view of the steps in Example 1.
Figure 2 is a schematic view of the steps in Example 3.
Figure 3 is a view showing a change over time in acid value (AV) in the esterification reaction of Step C (change over time after raising the temperature).

Best Mode for Carrying Out the Invention

As the carboxylic acid mixture that is a raw material of the present invention, it is possible to use an extract with water or an organic solvent of a by-product in preparation of cyclohexanone by oxidation of cyclohexane with oxygen or an oxygen-containing gas, or an extract obtained by once carrying out alkaline extraction and neutralization of the by-product; then combining the generated aqueous layer and an extract obtained by extraction of the remaining organic layer with an aqueous inorganic salt solution; and extracting them with an organic solvent. Such an extract contains a mixture of carboxylic acids such as glutaric acid, adipic acid and 6-hydroxycaproic acid.

Examples of the method for oxidizing cyclohexane with oxygen or an oxygen-containing gas include a method described in Non-Patent Document 1. Specifically, cyclohexane can be oxidized by introducing oxygen or an oxygen-containing gas into a reactor containing cyclohexane and carrying out reaction under pressure at 0.8 to 1.2 MPa at 150 to 180˚C using a salt of a metal such as cobalt (e.g. cobalt octylate) as a catalyst.

When the carboxylic acid mixture is extracted with water from the resulting oxidation reaction mixture, the amount of water is usually 1 to 10 wt% based on the amount of the oxidation reaction mixture.

The extracted aqueous layer generally contains 1 to 4 wt% of adipic acid, 1 to 4 wt% of 6-hydroxycaproic acid, 0.1 to 1 wt% of glutaric acid, 0.1 to 1 wt% of 5-hydroxyvaleric acid, 0.1 to 0.5 wt% of 1,2-cyclohexanediol (cis and trans), 0,1 to 0.5 wt% of 1,4-cyclohexanediol (cis and trans), 0.2 to 1 wt% of formic acid, and many other mono- and dicarboxylic acids, esters and oxo and oxa compounds, the content of each of which is generally not more than 0.5 wt%. Examples of the other mono- and dicarboxylic acids, esters and oxo and oxa compounds include acetic acid, propionic acid, butyric acid, valeric acid, caproic acid, oxalic acid, malonic acid, succinic acid, 4-hydroxybutyric acid and γ-butyrolactone.

The aqueous layer containing the carboxylic acid mixture is concentrated. The concentration is usually carried out by distillation. The aqueous layer is concentrated to 1/50 to 1/2 time, and preferably 1/20 to 1/3 time of the aqueous layer before the concentration on weight basis by distillation at a temperature of 10 to 250°C, preferably 20 to 200°C, and more preferably 30 to 200°C at a pressure of 0.1 to 150 KPa, preferably 0.5 to 110 KPa, and more preferably 2 to 100 KPa. The amount of water can be reduced to 2 wt% or less, and preferably 1 wt% or less based on the total amount by this concentration.

The oxidation reaction mixture of cyclohexane is saponified and the resulting alkaline solution is neutralized to generate an aqueous layer and an organic layer separately. When the aqueous layer and an extract obtained by extracting the organic layer with an aqueous inorganic salt solution are combined and extracted with an organic solvent, an aqueous solution of an alkali metal hydroxide such as sodium hydroxide is used as an alkali for the saponification. The concentration of the aqueous alkali metal hydroxide solution is 5 to 40 wt%, and the amount of the aqueous solution used is usually 1 to 2 moles per mole of the acids.

The resulting alkaline solution generally contains salts of adipic acid, 6-hydroxycaproic acid, glutaric acid, 5-hydroxyvaleric acid, formic acid and various other mono- and dicarboxylic acids such as acetic acid, propionic acid, butyric acid, valeric acid, caproic acid, oxalic acid, malonic acid, succinic acid and 4-hydroxybutyric acid, γ-butyrolactone, 1,2-cyclohexanediol (cis and trans), 1,4-cyclohexanediol (cis and trans) and the like.

The alkaline solution is neutralized with a mineral acid (such as sulfuric acid) to pH 3 or less, and preferably pH 2.5 or less. The alkaline solution is previously concentrated or the concentration of the mineral acid added is adjusted, so that the inorganic salt concentration in the aqueous layer at this time is 15 wt% or more, and preferably 20 wt% or more. Thus, the two layers, i.e. the aqueous layer and the organic layer, are separated.

The aqueous layer mainly contains adipic acid, 6-hydroxycaproic acid and their oligomers. When the inorganic salt concentration in the aqueous layer is 15 wt% or less, the aqueous layer contains an increased amount of monobasic acids which cannot be effective components of the desired 1,6-hexanediol and/or 1,5-pentanediol.

The organic layer also contains adipic acid, 6-hydroxycaproic acid and their oligomers. This is extracted with an aqueous inorganic salt solution having an inorganic salt concentration of 15 wt% or more, and more preferably 20 wt% or more. Examples of the aqueous inorganic salt solution include an aqueous mirabilite solution. The amount of the aqueous inorganic salt solution is 1 to 10 times the amount of the organic layer.

The aqueous layer and the extract of the organic layer with the aqueous inorganic salt solution are combined and extracted with an organic solvent such as methyl isobutyl ketone. The amount of the organic solvent used is 1/10 to 2 times the amount of the mixed solution obtained by combining the aqueous layer and the extract of the organic layer with the aqueous inorganic salt solution.

The extracted organic layer generally contains 2 to 10 wt% of adipic acid, 2 to 10 wt% of 6-hydroxycaproic acid, 0.1 to 2 wt% of glutaric acid, 0.1 to 2 wt% of 5-hydroxyvaleric acid, 0.1 to 1 wt% of 1,2-cyclohexanediol (cis and trans), 0.1 to 1 wt% of 1,4-cyclohexanediol (cis and trans), 0.2 to 2 wt% of formic acid and many other mono- and dicarboxylic acids, esters and oxo and oxa compounds, the content of each of which is generally not more than 1 wt%. Examples of the other mono- and dicarboxylic acids, esters and oxo and oxa compounds include acetic acid, propionic acid, butyric acid, valeric acid, caproic acid, oxalic acid, malonic acid, succinic acid, 4-hydroxybutyric acid and γ-butyrolactone.

The organic layer containing the carboxylic acid mixture is concentrated. The concentration is usually carried out by distillation. The content of the organic solvent can be reduced to 5 wt% or less, and preferably 1 wt% or less by distillation at a temperature of 10 to 250°C, preferably 20 to 225°C, and more preferably 30 to 200°C at a pressure of 0.1 to 150 KPa, preferably 0.5 to 110 KPa, and more preferably 2 to 100 KPa.

An alcohol is used for esterification of the carboxylic acid mixture obtained by extracting with water a reaction mixture obtained by oxidation of cyclohexane with oxygen or an oxygen-containing gas; or saponifying the reaction mixture and then neutralizing the resulting alkaline solution; and extracting with an organic solvent the generated aqueous layer and an extract obtained by extracting the organic layer with an aqueous inorganic salt solution. Examples of the alcohol include linear or branched aliphatic alcohols having 1 to 6 carbon atoms. Specific examples of the alcohol include monohydric alcohols such as methanol, ethanol, propanol, butanol, pentanol and hexanol; dihydric alcohols such as 1,6-hexanediol, ethylene glycol, propylene glycol, 1,4-butanediol, 1,5-hexanediol, diethylene glycol, 1,2-diols (such as 1,2-ethanediol and 1,2-propanediol) and 1,3-diols (such as 1,3-propanediol); and alicyclic alcohols such as cyclohexanol.

From the viewpoint of simplifying the separation and purification process, it is preferable to use 1,5-pentanediol and/or 1,6-hexanediol prepared by the present invention as the alcohol. From the viewpoint of separating the excess alcohol after the esterification, methanol, ethanol, propanol and butanol are preferable.

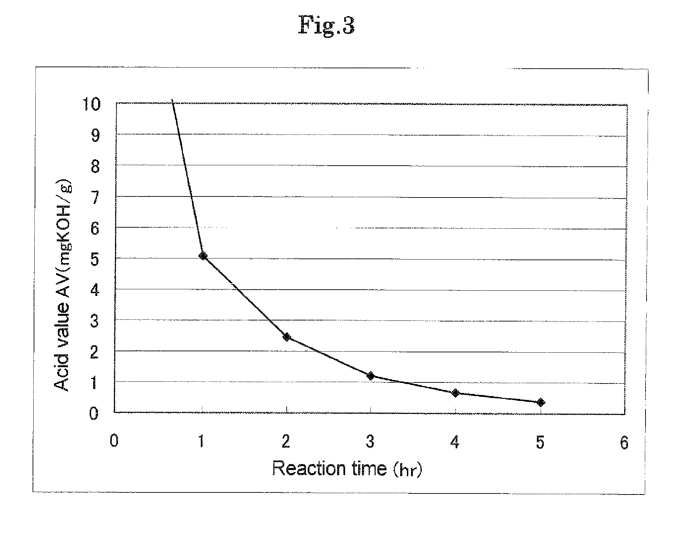

The completeness of esterification is verified by the acid value (AV) of the resulting esterified product. Usually, the acid value must be reduced to 1 mg KOH/g or less, and preferably 0.5 mg KOH/g or less, to inhibit poisoning of the copper-containing catalyst used in the next hydrogenation step. However, the esterification must be allowed to proceed at a high conversion rate to reduce the acid value to 0.5 mg KOH/g or less. That is, taking into consideration that the above carboxylic acid mixture concentrate generally has an acid value of 300 to 500 mg KOH/g, the conversion rate must be 99.8% or more to reduce the acid value to 0.5 mg KOH/g or less. This involves too much difficulty. For example, as Figure 3 shows a change over time of the acid value (AV) in esterification in Step 3 of Example 3 (change over time after raising the temperature), it may take only about 1.5 hours to reduce the acid value to 4 mg KOH/g or less; however, it takes five hours or more to reduce the acid value to 0.5 mg KOH/g or less. The esterification in Examples is carried out batchwise and in a short time; however, it takes a longer time to carry out continuous esterification.

When the resulting esterified product is an ester of a low-boiling primary alcohol such as methanol, ethanol, propanol or butanol, the esterified product can be purified by a conventional method such as distillation. However, when the acid components have a boiling point near the boiling point of the ester, it is difficult to use common equipment, or it is necessary to use complicated equipment and high energy, to reduce the acid value to 0.5 mg KOH/g or less. For example, even when purification was carried out by simple distillation after esterification with methanol as in Example 1, the acid value (AV) was 1 mg KOH/g or more.

On the other hand, when the alcohol is a secondary alcohol such as 1,5-hexanediol or 1,6-hexanediol, most of the ester exists as a dimer or oligomer and thus the acid value cannot be reduced by distillation purification.

A method of neutralizing the remaining organic acids with an alkali is also possible as a method for reducing the acid value; however, the process becomes complicated and there is a concern that the hydrogenation reaction step and the following steps may be adversely affected.

Since a copper-containing catalyst reduced (prereduced) in an alcohol having an acid value (AV) of 0.5 mg KOH/g or less or an ester having the same acid value is used in the present invention, the acid value (AV) of the esterified product is not particularly limited but is preferably 4 mg OH/g or less, and more preferably 0.5 to 1.5 mg KOH/g. The later-described esterification conditions are usually realized industrially. Although it is difficult to reduce the acid value (AV) of the resulting esterified product to 0.5 mg KOH/g or less as described above, it is relatively easy to reduce the acid value to 4 mg KOH/g or less. In the present invention, since a copper-containing catalyst is reduced (prereduced) in an alcohol having an acid value (AV) of 0.5 mg KOH/g or less or an ester having the same acid value, it is possible to inhibit aggregation of copper which easily occurs during reduction. Therefore, even when the acid value in the esterified product is 0.5 mg to 4 mg KOH/g, poisoning by the acids can be inhibited and the hydrogenation yield is improved. Further, the catalyst life is extended when the hydrogenation reaction is a fixed bed type, and filterability is improved when the hydrogenation reaction is a suspended bed type.

With regard to the amount of the alcohol used in the esterification step, the mixing ratio of the alcohol to the above concentrated carboxylic acid mixture (COA) (mass ratio) is 0. to 30, advantageously 0.2 to 20, and particularly advantageously 0.5 to 10.

The esterification is carried out by bringing the above concentrated carboxylic acid mixture (COA) into contact with the alcohol in a reaction vessel such as a stirring tank, a reaction tube, a bubble column or a distillation column or using a plurality of such reaction vessels as necessary. In the esterification reaction, the generated water is preferably removed from the reaction system. In this case, water can be distilled off together with an alcohol when the alcohol is a low-boiling alcohol such as methanol, ethanol, propanol or butanol, or water can be distilled off together with an inert gas such as nitrogen when the alcohol is a high-boiling alcohol such as 1,5-pentanediol or 1,6-hexanediol.

In this case, not all carboxyl groups present in the system are not present as an ester of the alcohol used. Some of the carboxyl groups may be present as a dimer or oligomer ester with the OH-terminal group of hydroxy caproic acid, for example.

In order to increase the esterification yield, an alcohol and the later-described esterification catalyst can also be added to the reaction solution after the esterification or its distillation residue to make the esterification further proceed in a tank or tube reactor.

The heating temperature in the esterification reaction can be appropriately selected according to the type of the alcohol used. The esterification reaction can be carried out at a temperature of 50 to 400˚C, preferably 70 to 300˚C, and more preferably 90 to 250˚C.

The esterification reaction can be carried out under reduced pressure conditions or under pressure conditions, or can be carried out under self-pressure in the esterification reactor. The esterification reaction is preferably carried out under pressure at 0 to 5 MPa, in particular, 0 to 2 MPa.

The reaction time in the esterification reaction can be appropriately selected according to the type of the alcohol used, the amount of the reaction raw material (carboxylic acid mixture), the catalyst and the like, but must be selected so that the esterified product has an acid value (AV) of 4 mg KOH/g or less. The reaction time can be 0.3 to 20 hours, for example, and preferably 0.5 to 10 hours.

The esterification reaction can be carried out without addition of a catalyst, but can also be carried out in the presence of a catalyst to increase the reaction rate. A homogeneously dissolved catalyst or a solid catalyst can be used as the catalyst. Examples of the homogeneously dissolved catalyst include mineral acids (such as sulfuric acid, phosphoric acid and hydrochloric acid), sulfonic acids (such as p-toluenesulfonic acid), heteropolyacids (such as phosphotungstic acid) and Lewis acids (such as an aluminum compound, a vanadium compound, a titanium compound, a boron compound and a zinc compound).

An acidic or peracidic material can be used as the solid catalyst. Examples of the material include acidic or peracidic metal oxides, for example, metal oxides such as $SiO_2$, $Al_2O_3$, $SnO_2$, $ZrO_2$, a layered silicate and zeolite to which a mineral acid residue such as a sulfate group or a phosphate group is added to increase the acidity; and organic ion exchangers having a sulfonic acid group or a carboxylic acid group. The solid catalyst can be used as a fixed bed or as a suspended bed.

In the case of the suspended bed, the amount of the catalyst used is 0.1 to 5 wt% based on the total amount. In the case of the fixed bed, the LHSV (liquid hourly space velocity) is in the range of 0.1 to 5 h$^{-1}$.

The amount of the homogeneously dissolved catalyst or the solid catalyst used is 0.01 to 1 wt% based on the total amount.

The copper-containing catalyst used for hydrogenating the esterified product is previously reduced (prereduced) in an alcohol having an acid value (AV) of 0.5 mg KOH/g or less or/and an ester having the same acid value. There are no particular limitations to the alcohol having an acid value (AV) of 0.5 mg KOH/g or less or/and the ester having the same acid value. However, the alcohol is advantageously an alcohol used for the esterification. Examples of the alcohol include methanol, ethanol, propanol, butanol, 1,5-hexanediol, 1,6-hexanediol and a mixture thereof Examples of the ester include the above esterified product of carboxylic acids obtained by oxidation of cyclohexane. Advantageously, it is possible to use a step solution in the purification step after the hydrogenation reaction of the esterified product (containing 1,6-hexanediol or/and 1,5-pentanediol).

The acid value (AV) of the alcohol having an acid value (AV) of 0.5 mg KOH/g or less or the ester having the same acid value can be adjusted by neutralization or/and distillation purification. The acid value (AV) of the ester can also be adjusted by further esterification. In the above conventional method, it is necessary to adjust the acid value (AV) of the whole of the esterified product to be hydrogenated. However, in the present invention, it is enough to adjust only the acid value of a small amount of the ester or alcohol used for reduction of catalyst, and it is not necessary to adjust the whole of the esterified product to be hydrogenated. In particular, when the ester is used for reduction of catalyst, the acid value is easily adjusted, since the ester whose acid value is easily adjustable may be used.

Examples of the copper-containing catalyst include simple copper oxide as well as a copper-zinc oxide catalyst, a copper-chromium oxide catalyst and a copper-zinc-titanium oxide catalysts. Desirably, the content of copper oxide in the catalyst is 5 to 98 wt%, preferably 20 to 98 wt%, and particularly preferably 20 to 80 wt% based on the total weight of the catalyst. The catalyst may contain a small amount of a metal such as aluminum, magnesium or zirconium.

The amount of the copper-containing catalyst used is 10 to 300 g, and preferably 50 to 200 g based on 1 L of the alcohol having an acid value (AV) of 0.5 mg KOH/g or less or/and the ester having the same acid value.

The prereduction temperature is preferably as low as possible to the extent that reduction occurs. The prereduction temperature is not limited but is preferably 110 to 150°C, and more preferably 120 to 140°C.

The hydrogen pressure in the Prereduction is preferable as high as possible, but is 0.1 to 30 MPa, and preferably 0.3 to 2 MPa from a practical standpoint.

The catalyst may be prereduced continuously or batchwise. When the catalyst is powder, the reaction form may be a normal gas-liquid-solid reactor such as a gas-liquid-solid stirring tank, a suspended bubble column or a tube reactor. When the catalyst is a molded article such as a pellet, a fixed bed reactor is packed with the catalyst, and the alcohol having an acid value (AV) of 0.5 mg KOH/g or less or/and the ester having the same acid value and hydrogen gas are circulated in the reactor.

The crystallite size of copper(0) of the copper-containing catalyst after the prereduction can be measured by X-ray diffractometry (XRD). The crystallite size is calculated by the following Scherrer's formula from the diffraction line broadening (half width) using Cu(111) for the peak diffraction angle 2θ.

$$D = K \cdot \lambda / (\beta \cdot \cos\theta)$$

D: Crystallite size (Å)
K: Scherrer constant (0.94)
λ: Measured X-ray wavelength (1.5406 A : CuKα1)
β: Diffraction line broadening based on crystallite size (rad.)
θ: Bragg angle (diffraction angle/2)

As shown in the later-described Table 5, the copper-containing catalyst of the present invention after the prereduction has been found to have a crystallite size of copper(0) of 150 Å or less which is smaller than that of the catalyst not prereduced.

In the subsequent hydrogenation step, as the copper-containing catalyst has a smaller crystallite size, the activity and the hydrogenation yield of the catalyst are higher. That is, the prereduction can reduce the crystallite size of copper(0) of the copper-containing catalyst to 150 Å or less, preferably 120 A or less, and more preferably 100 Å or less. As a result, the hydrogenation yield is improved.

The esterified product is hydrogenated using the copper-containing catalyst prereduced in the alcohol having an acid value (AV) of 0.5 mg KOH/g or less or/and the ester having the same acid value. Examples of the hydrogenation reaction form of the esterified product include a liquid phase suspension reaction form and a fixed bed catalyst reaction form.

When the esterified product is hydrogenated in a liquid phase suspension, the amount of the copper-containing catalyst reduced in the alcohol having an acid value (AV) of 0.5 mg KOH/g or less or the ester having the same acid value is 0.1 to 10 wt%, and preferably 0.3 to 5 wt% based on the amount of the esterified product, the hydrogenation reaction temperature is 150 to 300˚C, and preferably 200 to 290˚C, and the hydrogen pressure is 1 to 30 MPa, and preferably 15 to 30 MPa.

The esterified product is hydrogenated in a fixed bed by supplying the esterified product at an LHSV (liquid hourly space velocity) of 0.01 to 10 g/ml·h, and preferably 0.1 to 5 g/ml·h and supplying hydrogen gas at a GHSV (gas hourly space velocity) of 10 to 10000/hr, and preferably 100 to 3000/hr at a hydrogen pressure of 1 to 30 MPa, and preferably 5 to 20 MPa at a hydrogenation reaction temperature of 150 to 300˚C, and preferably 180 to 250˚C.

The esterified product of the present invention is usually hydrogenated in the absence of a solvent. However, it is also possible to use solvents such as all inert substances under the reaction conditions, for example, monohydric alcohols such as methanol, ethanol, propanol, butanol, pentanol and hexanol; dihydric alcohols such as 1,6-hexanediol, ethylene glycol, propylene glycol, 1,4-butanediol, 1,5-hexanediol and diethylene glycol; alicyclic alcohols such as cyclohexanol; ethers such as tetrahydrofuran and ethylene glycol ether; and hydrocarbons such as hexane.

1,5-pentanediol and/or 1,6-hexanediol that are the desired products in the reaction solution obtained by separating the catalyst after the hydrogenation can be separated and purified by a conventional method such as distillation.

When 1,5-pentadiol and 1,6-hexanediol are prepared, the distillation is carried out by distilling off low-boiling substances under reduced pressure of 1.33 to 26.6 kPa (10 to 200 torr) at a reflux ratio of 0.1 to 30, then distilling off a fraction containing 1,5-pentanediol as a main component at 0.133 to 13.3 kPa (1 to 100 torr) at a reflux ratio of 0.5 to 30, and finally distilling off a fraction containing 1,6-hexanediol as a main component at 0.133 to 13.3 kPa (1 to 100 torr) at a reflux ratio of 0.10 to 10, for example.

A low-boiling substance or a column bottom liquid containing an unreacted carboxylic acid component and its ester and the like can be recycled as a raw material for hydrogenation.

The acid value (AV) in the Examples was the weight (mg) of KOH (potassium hydroxide) necessary for neutralizing a sample of unit amount (1 g) and was determined by titration.

The saponification value (SV) in the Examples was the weight (mg) of KOH (potassium hydroxide) necessary for saponifying a sample of unit amount (1 g) and was determined by neutralization titration (back titration) of the excess KOH after saponification with hydrochloric acid.

Each carboxylic acid component contained in the carboxylic acid mixture was determined by gas chromatographic analysis or liquid chromatographic analysis.

The alcohol such as 1,6-hexanediol generated by hydrogenation reaction of the esterified product was determined by gas chromatographic analysis.

In the filterability test, 120 ml of the slurry after the hydrogenation reaction was placed in a pressure filtration device, and the time until the amount of the filtrate was changed from 50 ml to 100 ml was measured when filtration was carried out at room temperature while maintaining a pressure of 0.05 MPaG (0.3 MPaG in Examples 3 to 10 and Comparative Examples 2 to 7) with nitrogen.

[Examples]

The present invention will be described in detail below with reference to Examples.

[Example 1]

Step 1: Oxidation of cyclohexane and extraction with water

Cyclohexane was oxidized under the conditions of 160˚C and 1 MPa and extracted with water under the conditions of 160˚C and 1 MPa to obtain a carboxylic acid mixture having the following composition.

<Composition of water extract of cyclohexane oxidation>

Valeric acid: 0.2%
Caproic acid: 0.03%
Succinic acid: 0.2%
6-Hydroxycaproic acid: 5.5%
Glutaric acid: 0.7%
Adipic acid: 4.5%
Water ($H_2O$): 78.3%
Others: 10.57%

Step 2: Concentration of water extract

Then, the extract was concentrated under the condition of 13 KPa to obtain a concentrate having the following composition.

6-Hydroxycaproic acid: 28.66%
Adipic acid: 23.80%
$H_2O$: 2.0%

Step 3: Esterification

700 g/h of the bottom liquid (the above concentrate) obtained in Step 2 and 700 g/h of methanol are continuously fed to reaction tanks (two gas-liquid reaction tanks) to carry out esterification.

Reaction tank conditions: 1 MPa, 240˚C, retention time: 1 hour (per tank) $\times$ 2 tanks
Gas side -> 756 g/h, composition of concentrate on the gas side: $H_2O$ = 6.8%, dimethyl adipate = 10.5%, methyl 6-hydroxycaproate = 1.8%
Bottom side -> 644 g/h, acid value = 20 mg KOH/g, $H_2O$ = 0.1%
(The most of adipic acid and 6-hydroxycaproic acid was present as oligomers.)

Step 4: Fractional distillation of carboxylate

Methanol was recovered from the distillate from Step 3 (esterification step) in a first column and water and the low-boiling component were removed from the distillate in a second column under the following conditions.

First column

Distillation apparatus: Sulzer Labopacking (x 5)
Distillation conditions: Normal pressure, column top 65˚C, column bottom 111˚C

Second column

Distillation apparatus: Sulzer Labopacking ($\times$ 5)
Distillation conditions: 410 Torr, column top 76˚C, column bottom 190˚C

Step 5: Depolymerization

100 g/h of the bottom liquid obtained in Step 3, 200 g/h of methanol and 0.1 g/h of a tetrabutoxytitanium catalyst were continuously fed to a tube reactor to carry out depolymerization reaction under the following conditions.

Reactor conditions: 270˚C, 10 MPa, retention time: 5 minutes

Step 6: Removal of methanol

The reaction solution obtained by the depolymerization in Step 5 was distilled under the following conditions to remove methanol and the low-boiling component.

Distillation apparatus: Sulzer Labopacking ($\times$ 5)
Distillation conditions: 160 Torr, column top 34˚C, column bottom 89˚C
The bottom liquid had an acid value of 2.0 mg KOH/g.

Step 7: Purification of ester

The catalyst and the high-boiling component were removed from the bottom liquids obtained in Steps 4 and 6 by simple distillation at a pressure of 1.3 kPa to obtain an esterified product containing dimethyl adipate and methyl oxycaproate as main components. The esterified product had an acid value of 1.1 mg KOH/g.

Step 8: Prereduction of catalyst

130 g of 1,5-pentanediol (manufactured by Ube Industries, Ltd.) (AV 0.01 mg KOH/g, water content 100 ppm) and 12 g of a Cu-Zn composite oxide catalyst [prepared by the method described in Example 1 of Patent Document 6] were placed in a 200 cc autoclave made of stainless steel. The temperature was raised to 130˚C over 30 minutes while supplying hydrogen so that the pressure was 1 MPa. Thereafter, reduction was carried out for one hour. After the reaction, the catalyst was separated by centrifugation and used for the next hydrogenation reaction. The result of measuring the crystallite size of copper(0) of the catalyst by XRD is shown in Table 5.

Step 9: Hydrogenation of esterified product

1200 g of the esterified product in Step 7 and 12 g of the catalyst prereduced in Step 8 were placed in an autoclave made of stainless steel. Hydrogenation reaction was carried out at 275˚C for 1.5 hours while supplying hydrogen so that the pressure was 25 MPa. After the reaction, the catalyst was filtered to obtain 1220 g of a filtrate. The SV conversion rate of the filtrate was 86.6%. The filtration rate according to the filterability test was 102 seconds.

Step 10: Purification of 1,6-hexanediol

800 g of the filtrate was placed in a 1000 ml flask having a bottom to which a 25 $\phi$ packed column made of glass (packing material: Sulzer Labopack 100 cm) was attached. 241.6 g of the low-boiling substance was distilled off at a reflux ratio of 5 under reduced pressure of 13.3 kPa (100 Torr). Further, 510.4 g of a main fraction containing 1,6-hexanediol as a main component and 34.7 g of a column bottom liquid were obtained at 1.33 kPa (10 Torr) at a reflux ratio of 5.

The main fraction contained 0.2 wt% of dimethyl adipate, 2.0 wt% of methyl 6-hydroxycaproate, 2.9 wt% of ε-caprolactone, 11.4% of 1,5-pentanediol and 81.4 wt% of 1,6-hexanediol. 1,6-hexanediol having a gas chromatographic purity of 99.8% or more could be obtained by distilling the main fraction.

The column bottom liquid contained effective components such as 1,6-hexanediol, an ester of 6-hydroxycaproic acid and 1,6-hexanediol, and an ester of adipic acid and 1,6-hexanediol and thus could be recycled as a raw material for esterification reaction or hydrogenation reaction.

[Example 2]

Part of the bottom liquids obtained in Steps 4 and 6 of Example 1 was distilled and purified under the following conditions to obtain a fraction containing methyl adipate and methyl 6-hydroxycaproate as main components from the column top. At that time, the acid value was 0.1 mg KOH/g.

Distillation apparatus: Sulzer Labopacking ($\times$ 27)
Distillation conditions: 5 Torr, column top 105˚C, column bottom 190˚C

The catalyst prereduction in Step 8 was carried out by the same manner, except for using the above purified methyl ester as a solvent. Thereafter, hydrogenation reaction was carried out in the same manner as in Step 9. The results at that time are shown in Table 1. The result of measuring the crystallite size of copper(0) of the catalyst by XRD is shown in Table 5.

[Comparative Example 1]

Hydrogenation was carried out under the same conditions as in Step 9 using the catalyst described in Step 8 directly in Step 9 without carrying out the catalyst prereduction in Step 8. The results at that time are shown in Table 1.

Table 1

| | Prereduction solvent | | Hydrogenation reaction results | |
|---|---|---|---|---|
| | Name of solvent | AV (mgKOH/g) | SV conversion rate (%) | Filtration rate (sec) |
| Example 1 | 1,5-Pentanediol | 0.01 | 86.6 | 102 |
| Example 2 | Purified ester | 0.1 | 90.6 | 91 |
| Comparative Example 1 | Without prereduction | | 79.8 | 323 |

[Example 3]

Step A: Oxidation of cyclohexane and extraction with water

Cyclohexane was oxidized under the conditions of 160°C and 1 MPa and extracted with water under the conditions of 160°C and 1 MPa to obtain a carboxylic acid mixture having the following composition.

<Composition of water extract of cyclohexane oxidation>

Valeric acid: 0.2%
Caproic acid: 0.03%
Succinic acid: 0.2%
6-Hydroxycaproic acid: 5.5%
Glutaric acid: 0.7%
Adipic acid: 4.5%
$H_2O$: 78.3%
Others: 10.57%

Step B: Concentration of water extract

Then, the extract was concentrated under the condition of 13 KPa to obtain a concentrate having the following composition.

6-Hydroxycaproic acid: 28.66%
Adipic acid: 23.80%
$H_2O$: 2.0%

Step C: Esterification step

500 g of the above concentrate and 500 g of the filtrate of the hydrogenation reaction solution in Step 9 of Example 1 were placed in a 1 L flask. The temperature was raised to 250°C while bubbling nitrogen gas at a rate of 200 cc/minute, and maintained at that temperature for 3.5 hours. During that time, only the aqueous layer of the distillate was extracted from the system by a Dean-Stark apparatus, and the organic layer of the distillate was refluxed to the flask. After a predetermined time had elapsed, the temperature was fallen down and the reaction was terminated. The bottom liquid remaining in the flask (diol ester) had a weight of 903 g and an acid value of 0.9 mg KOH/g.

Step D: Prereduction of catalyst

130 g of 1,5-pentanediol (manufactured by Ube Industries, Ltd.) (acid value (AV) 0.01 mg KOH/g, water content 100 ppm) and 12 g of a Cu-Zn composite oxide catalyst [prepared by the method described in Example 1 of Patent Document 6] were placed in a 200 cc autoclave made of stainless steel. After replacement with hydrogen, the pressure was raised to 1 KPa. The temperature was raised to 130°C over 30 minutes while supplying hydrogen so that the pressure was 1 MPa. Thereafter, reduction was carried out for one hour. After the reaction, the catalyst was separated by centrifugation and used for the next hydrogenation reaction. The result of measuring the crystallite size of copper(0) of the catalyst by XRD is shown in Table 5.

Step E: Hydrogenation of esterified product

1200 g of the esterified product in Step C and 12 g of the catalyst prereduced in Step D were placed in an autoclave made of stainless steel.
Hydrogenation reaction was carried out at 275°C for 1.5 hours while supplying hydrogen so that the pressure was 25 MPa. After the reaction, the catalyst was filtered to obtain 1220 g of a filtrate. The SV conversion rate of the filtrate was 87.4%. The filtration rate according to the filterability test was 221 seconds.

Step F: Purification of 1,6-hexanediol

800 g of the filtrate was placed in a 1000 ml flask having a bottom to which a 25 $\phi$ packed column made of glass (packing material: Sulzer Labopack 100 cm) was attached. 41.5 g of the low-boiling substance was distilled off at a reflux ratio of 5 under reduced pressure of 13.3 kPa (100 Torr). Further, 453.4 g of a main fraction containing 1,6-hexanediol as a main component and a column bottom liquid were obtained at 1.33 kPa (10 Torr) at a reflux ratio of 5.
The main fraction contained 82.6 wt% of 1,6-hexanediol, 9.6 wt% of 1,5-pentanediol and 2.0 wt% of 1-hexanol, and additionally contained 1,4-cyclohexanediol, 1,2-cyclohexanediol, 1,5-hexanediol and the like. 1,6-Hexanediol having a gas chromatographic purity of 98% or more could be obtained by distilling the main fraction.

[Comparative Example 2]

Hydrogenation was carried out in the same manner as in Step E using the catalyst described in Step D directly in Step E without carrying out the catalyst prereduction in Step D of Example 3. The results at that time are shown in Table 2.

[Example 4]

Example 4 was carried out in the same manner as in Example 3, except that caproic acid was added to 1,5-pentanediol to adjust the AV to 0.27 mg KOH/g for the catalyst prereduction in Step D of Example 3. The results at that time are shown in Table 2.

[Example 5]

Example 5 was carried out in the same manner as in Example 3, except that caproic acid was added to 1,5-pentanediol to adjust the AV to 0.50 mg KOH/g for the catalyst prereduction in Step D of Example 3. The results at that time are shown in Table 2.

[Comparative Example 3]

Comparative Example 3 was carried out in the same manner as in Example 3, except that caproic acid was added to 1,5-pentanediol to adjust the AV to 1.03 mg KOH/g for the catalyst prereduction in Step D of Example 3. The results at that time are shown in Table 2.

[Comparative Example 4]

Comparative Example 4 was carried out in the same manner as in Example 3, except that caproic acid was added to 1,5-perntanediol to adjust the AV to 1.59 mg KOH/g for the catalyst prereduction in Step D of Example 3. The results at that time are shown in Table 2.
Table 2

| | Prereduction solvent | | Hydrogenation reaction results | |
|---|---|---|---|---|
| | Name of solvent | AV (mgKOH/g) | SV conversion rate (%) | Filtration rate (sec) |
| Comparative Example 2 | Without prereduction | | 79.5 | 2288 |
| Example 3 | 1,5-pentanediol | 0.01 | 87.4 | 221 |
| Example 4 | 1,5-Pentanediol | 0.27 | 83.9 | 191 |
| Example 5 | 1,5-pentanediol | 0.50 | 83.8 | 214 |
| Comparative Example 3 | 1,5-pentanediol | 1.03 | 71.5 | 1865 |
| Comparative Example 4 | 1,5-Pentanediol | 1.59 | 45.9 | >3600 |

[Example 6]

Example 6 was carried out in the same manner as in Example 3, except that the time for the esterification reaction in Step C of Example 3 was reduced so that the AV was 1.8 mg KOH/g. The results are shown in Table 3.

[Example 7]

Example 7 was carried out in the same manner as in Example 3, except that the time for the esterification reaction in Step C of Example 3 was reduced so that the AV was 2.7 mg KOH/g. The results are shown in Table 3.

[Example 8]

Example 8 was carried out in the same manner as in Example 3, except that the time for the esterification reaction in Step C of Example 3 was reduced so that the AV was 3.4 mg KOH/g. The results are shown in Table 3.

[Example 9]

Example 9 was carried out in the same manner as in Example 3, except that the time for the esterification reaction in Step C of Example 3 was reduced so that the AV was 4.0 mg KOH/g. The results are shown in Table 3.

[Example 10]

Example 10 was carried out in the same manner as in Example 3, except that the time for the esterification reaction in Step C of Example 3 was reduced so that the AV was 8.3 mg KOH/g. The results are shown in Table 3.
Table 3

| | Prereduction | Esterified product | Hydrogenation reaction results | |
|---|---|---|---|---|
| | | AV (mgKOH/g) | SV conversion rate (%) | Filtration rate (sec) |
| Comparative Example 2 | No | 0.9 | 79.5 | 2288 |
| Example 3 | Yes | 0.9 | 87.4 | 221 |
| Example 6 | Yes | 1.8 | 86.6 | 827 |
| Example 7 | Yes | 2.7 | 85.4 | 1108 |
| Example 8 | Yes | 3.4 | 85.6 | 986 |
| Example 9 | Yes | 4.0 | 84.3 | 1287 |
| Example 10 | Yes | 8.3 | 60.7 | 3400 |

[Comparative Examples 5 to 7]

Comparative Examples 5 to 7 were carried out in the same manner as in Example 3, except that catalyst prereduction was not carried out and the acid value (AV) of the esterified product was changed. The results are shown in Table 4.

Table 4

| | Prereduction | Esterified product | Hydrogenation reaction results | |
|---|---|---|---|---|
| | | AV (mgKOH/g) | SV conversion rate (%) | Filtration rate (sec) |
| Comparative Example 2 | No | 0.9 | 79.5 | 2288 |
| Comparative Example 5 | No | 1.8 | 50.9 | >3600 |
| Comparative Example 6 | No | 3.0 | 45.6 | >3600 |
| Comparative Example 7 | No | 8.0 | 30.0 | >3600 |

In order to examine the crystal size of copper(0) of the copper-containing catalyst before the hydrogenation reaction, the catalyst after the prereduction was taken out in Examples 1 to 5 and Comparative Examples 3 to 4 and the catalyst was taken out before the hydrogenation reaction in Comparative Examples 1 to 2 to carry out X-ray diffraction (XRD) analysis. The results are shown in Table 5.

Table 5

| | Prereduction solvent | Acid value of prereduction solvent (mg KOH/g) | Crystallite size of Cu(0) before hydrogenation reaction (after prereduction) (Å) |
|---|---|---|---|
| Example 1 | 1,5-Pentanediol | 0.01 | 63 |
| Example 2 | Methyl ester | 0.1 | 105 |
| Comparative Example 1 | Without prereduction | | 280 |
| Comparative Example 2 | Without prereduction | | 170 |
| Example 3 | 1,5-pentanediol | 0.01 | 63 |
| Example 4 | 1,5-Pentanediol | 0.27 | 68 |
| Example 5 | 1,5-Pentanediol | 0.50 | 72 |
| Comparative Example 3 | 1.5-Pentanediol | 1.03 | 160 |
| Comparative Example 4 | 1,5-Pentanediol | 1.59 | 178 |

Industrial Applicability

According to the present invention, industrially useful 1,5-pentanediol and/or 1,6-hexanediol can be prepared in a high yield while controlling deterioration of a hydrogenation catalyst.

**Claims**

1. A process for preparing 1,5-pentanediol and/or 1,6-hexanediol, comprising esterifying monocarboxylic and dicarboxylic acids which are by-products in preparation of cyclohexanone by oxidation of cyclohexane; and hydrogenating the resulting esterified product in the presence of a copper-containing catalyst reduced (prereduced) in the presence of an alcohol having an acid value of 0.5 mg KOH/g or less or/and an ester having the same acid value.

2. The process for preparing 1,5-pentanediol and/or 1,6-hexanediol according to claim 1, wherein the esterified product has an acid value of 4 mg KOH/g or less.

3. The process for preparing 1,5-pentanediol and/or 1,6-hexanediol according to claim 1, wherein the alcohol is an aliphatic alcohol having 1 to 6 carbon atoms.

4. The process for preparing 1,5-pentanediol and/or 1,6-hexanediol according to claim 1, wherein the copper-containing catalyst is a catalyst containing copper oxide or a composite metal oxide composed of copper oxide and zinc oxide.

5. The process for preparing 1,5-pentanediol and/or 1,6-hexanediol according to claim 1, wherein the copper(0) of the reduced (prereduced) copper-containing catalyst has a crystallite size of 150 Å or less after reduction.

6. A copper-containing catalyst for hydrogenation wherein the copper(0) has a crystallite size of 150 Å or less.

## Fig.1

Recovered MeOH

Step 4
First column MeOH recovery

H2O

Step 4
Second column
Distillation off of water

Step 1
Water
extraction

Step 2
Concentration

Step 3
Esterification

Esterified product

Alcohol or ester

Recovered MeOH

Step 8
Prereduction of catalyst

Step 5
Depolymerization

Step 6
Distillation off
of MeOH

Step 9
Hydrogenation step

Step 7
Purification of ester

Step 10
Diol purification step

Bottom Residue

## Fig.2

Alcohol or ester

Step D
Prereduction
of catalyst

Step A
Water
extraction

Step B
Concent-
ration

Step C
Esterification

Step E
Hydrogenation
step

Step F
Diol purification step

Fig.3

**INTERNATIONAL SEARCH REPORT**

| International application No. |
|---|
| PCT/JP2008/058900 |

A. CLASSIFICATION OF SUBJECT MATTER
*C07C29/149*(2006.01)i

According to International Patent Classification (IPC) or to both national classification and IPC

B. FIELDS SEARCHED

Minimum documentation searched (classification system followed by classification symbols)
C07C29/149

Documentation searched other than minimum documentation to the extent that such documents are included in the fields searched
Jitsuyo Shinan Koho        1922-1996    Jitsuyo Shinan Toroku Koho    1996-2008
Kokai Jitsuyo Shinan Koho   1971-2008    Toroku Jitsuyo Shinan Koho    1994-2008

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | JP 7-233108 A (Ube Industries, Ltd.), 05 September, 1995 (05.09.95), & EP 661255 A1 & US 5536888 A | 1-6 |
| Y | JP 2000-505468 A (BASF AG.), 09 May, 2000 (09.05.00), & WO 97/31882 A1 & EP 883590 A1 & US 6008418 A | 1-6 |
| Y | JP 7-163880 A (Kao Corp.), 27 June, 1995 (27.06.95), (Family: none) | 1-6 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 03 June, 2008 (03.06.08) | 17 June, 2008 (17.06.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP SHO5333567 B
- JP HEI334946 B
- JP HEI5978 B
- JP 2005035974 A
- JP HEI7163880 B
- JP 3161578 B

**Non-patent literature cited in the description**

- Ullmann's Encyclopedia of Industrial Chemistry. 1987, vol. A8, 2, 9